# EUROPEAN PATENT SPECIFICATION

(11) **EP 3 843 627 B1**
(45) Date of publication and mention of the grant of the patent: **02.12.2026**
(21) Application number: 19856248.0
(22) Date of filing: 30.08.2019
(51) Int. Cl.: G06N 3/088, G06N 3/045, G06N 3/047, G06N 3/0895, G06N 3/09, G06T 12/20

(54) **APPLICATION OF MACHINE LEARNING TO ITERATIVE AND MULTIMODALITY IMAGE RECONSTRUCTION**
ANWENDUNG VON MASCHINELLEM LERNEN ZUR ITERATIVEN UND MULTIMODALEN BILDREKONSTRUKTION
APPLICATION D'APPRENTISSAGE MACHINE À UNE RECONSTRUCTION D'IMAGE ITÉRATIVE ET MULTIMODALITÉ

(30) Priority: 31.08.2018 US 201862725882 P
(43) Date of publication of application: 07.07.2021
(73) Proprietor: QT Imaging, Inc., Novato, CA 94949 (US)
(72) Inventor: LENOX, Mark Wayne, Novato, California 94949 (US); PIRSHAFIEY, Nasser Charles, Novato, California 94949 (US); CWIKLA, Martin, Novato, California 94949 (US); MALIK, Bilal, Novato, California 94949 (US); TIWARI, Sandeep, Novato, California 94949 (US)
(74) Representative: Forresters IP LLP
(86) International application number: PCT/US2019/049133
(87) International publication number: WO 2020/047456

(56) References cited:
- WO-A1-2017/223560
- WO-A1-2018/127498
- US-A1- 2010 183 217
- US-A1- 2015 003 708
- US-A1- 2015 279 060
- US-A1- 2017 200 265
- US-A1- 2017 309 019
- US-A1- 2018 018 757
- MORTEZA MARDANI ET AL: "Deep Generative Adversarial Networks for Compressed Sensing (GANCS) Automates MRI", 31 May 2017 (2017-05-31), pages 1 - 12, XP055593025, Retrieved from the Internet <URL:https://arxiv.org/pdf/1706.00051.pdf> [retrieved on 20190529]

## Description

### BACKGROUND

Medical imaging involves acquisition of data and image reconstruction from the data. Image reconstruction from acquired data can be treated as an inverse problem that can be solved via iterative methods. Often, an approximate solution - an assumed image or an initial estimate - is first obtained and then a better reconstruction is generated using multiple iteration steps. The iterative steps can include calculating the difference between the measured and the modeled fields utilizing all the acquired projections data, and updating the image based on comparison, resulting in progressive improvement of image quality and also reduction in reconstruction image artifacts. Image reconstruction may be carried out for imaging modalities such as magnetic resonance imaging (MRI), positron emission tomography (PET), computed tomography (CT), and ultrasound.

Ultrasound imaging uses high-frequency sound waves to view internal structures such as tissues and blood flow. A transducer transmits ultrasound waves and a receiver captures the waves after reflecting from or transmitting through the tissue. A quantitative transmission ultrasound system performs both transmission ultrasound and reflection ultrasound methods to gather data.
Morteza Mardani ET AL: "Deep Generative Adversarial Networks for Compressed Sensing (GANCS) Automates MRI" discloses magnetic resonance image (MRI) reconstruction is a severely ill-posed linear inverse task demanding time and resource intensive computations that can substantially trade off accuracy for speed in real-time imaging. In addition, state-of-the-art compressed sensing (CS) analytics are not cognizant of the image diagnostic quality. To cope with these challenges they put forth a CS framework that permeates benefits from generative adversarial networks (GAN) to train a (low-dimensional) manifold of diagnostic-quality MR images from historical patients. Leveraging a mixture of least-squares (LS) GANs and pixel-wise cost, a deep residual network with skip connections is trained as the generator that learns to remove the aliasing artifacts by projecting onto the manifold.
US2018018757 discloses a method and system for transforming low-quality projection data into higher quality projection data, using a machine learning model. Regions are extracted from an input projection image acquired, for example, at a reduced x-ray radiation dose (lower-dose), and pixel values in the region are entered into the machine learning model as input. The output of the machine learning model is a region that corresponds to the input region. The output information is arranged to form an output high-quality projection image. A reconstruction algorithm reconstructs high-quality tomographic images from the output high-quality projection images. The machine learning model is trained with matched pairs of projection images, namely, input lower-quality (lower-dose) projection images together with corresponding desired higher-quality (higher-dose) projection images. Through the training, the machine learning model learns to transform lower-quality (lower-dose) projection images to higher-quality (higher-dose) projection images.
WO2018127498 discloses an ultrasound imaging system that may include or be operatively associated with an ultrasound transducer configured to acquire echo signals responsive to ultrasound pulses transmitted toward a medium. The system may include a channel memory configured to store the acquired echo signals, a neural network coupled to the channel memory and configured to receive one or more samples of the acquired echo signals, one or more samples of beamformed signals based on the acquired echo signals, or both, and to provide imaging data based on the one or more samples of the acquired echo signals, the one or more samples of beamformed signals, or both, and may further include a display processor configured to generate an ultrasound image using the imaging data provided by the neural network.

### BRIEF SUMMARY

Aspects of the present invention are recited by the independent claims, with the dependent claims reciting optional features. Machine learning based image reconstruction is described. The application of machine learning to iterative and multimodality image reconstruction can be considered "efficient" image reconstruction due to the ability to reconstruct images with fewer data points of raw data. In the context of ultrasound imaging, efficient image reconstruction does not require the low frequency transmission ultrasound data in order to reconstruct transmission images such as speed of sound images. Rather, reflection data alone or, in examples not part of the claimed invention, in combination with transmission data obtained via more conventional ultrasound frequencies (e.g., from 0.8 MHz to 1.5 MHz in one instance) can be used to generate suitable transmission images.

An image reconstruction system includes a communication interface receiving imaging data from a scanner system and a reconstruction engine. The reconstruction engine includes two GAN-based neural networks, a processor, and storage media storing instructions for performing image reconstruction when executed by the processor. The neural networks include a GAN-based algorithm (resulting in a "GAN-based neural network", each) that receives at least a component of the imaging data from the scanner system via the communication interface.

The image reconstruction system can further include a repository that stores a training data set and/or images collected from use of an imaging modality system (e.g., quantitative transmission ultrasound systems) over time for training or self-learning of the neural network of the reconstruction engine.

In some cases, the system further includes a discriminator for use in training the one or more neural networks. For example, the repository can store a training set of reflection and transmission images and/or raw data The training or self-learning of the neural network can be accomplished by using the discriminator to compare the transmission data (corresponding to the reflection data) from the repository to the synthetic transmission data generated from the corresponding reflection data and output a set of errors that are used to adjust weights in the GAN-based algorithm for the neural network.

In some cases, one or more application programming interfaces can be provided as part of the image reconstruction system to support third party submission of training data and support third party submission of image reconstruction algorithms.

A method for image reconstruction includes receiving, at a reconstruction engine, imaging data; generating, using two GAN-based neural networks, intermediate synthetic transmission data from at least a component of the imaging data; performing iterative methods on at least the intermediate synthetic transmission data until a desired accuracy is reached; and upon reaching the desired accuracy, outputting final synthetic transmission data. When the imaging data includes reflection data, an initial/intermediate synthetic estimate for a transmission image can be generated using a neural network and GAN-based algorithm (a "GAN-based neural network") that is trained (machine or self-learning) on imaging data (e.g., paired reflection and transmission data such as available via quantitative transmission ultrasound); and an iterative method for image reconstruction is performed using the initial/intermediate synthetic estimate to generate increasingly accurate transmission images until a desired accuracy is reached. In some cases which are not part of the claimed invention, transmission data (e.g., of a conventional frequency range such as starting above 1.5 MHz) can be included with the received imaging data and the image reconstruction can be carried out using the transmission data and the initial/intermediate synthetic estimate. In the claimed invention, two types of reflection data (e.g., a reflection image(s) and a set of reflection data) are processed by GAN-based neural networks trained on the imaging data to create two types of initial/intermediate synthetic transmission data (e.g., a synthetic transmission image(s) and a set of synthetic transmission data). When only reflection data is received, the image reconstruction can be carried out using synthetic transmission data and the initial/intermediate synthetic estimate.

A method for training a neural network can include receiving training imaging data, the imaging data comprising paired transmission data and reflection data; generating, using a GAN-based neural network, synthetic transmission data from the reflection data; comparing transmission data that is paired with the reflection data with the synthetic transmission data to determine a set of errors; and adjusting weights of the GAN-based neural network using the set of errors. The method for training a neural network can further or alternatively include receiving paired transmission images and reflection images, generating using a GAN-based neural network, an initial guess of a synthetic transmission image from a reflection image; performing iterative methods using speed of sound data corresponding to a transmission image that is paired with the reflection image and the initial guess of the synthetic transmission image until a desired accuracy is reached; upon reaching the desired accuracy, outputting a final synthetic transmission image; comparing the transmission image that is paired with the reflection image with the final synthetic transmission image to determine a set of errors; and adjusting the weights of the GAN-based neural network using the set of errors.

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter.

### BRIEF DESCRIPTION OF THE DRAWINGS

**Figure 1** illustrates an operating environment for machine learning based image reconstruction.
**Figures 2A** **and** **2B** illustrate example reconstruction engine configurations.
**Figure 3** illustrates an example scenario using machine learning based image reconstruction.
**Figure 4** illustrates an example process for training a feature set of a neural network using paired data.

### DETAILED DISCLOSURE

The application of machine learning or self-learning to iterative and multimodality image reconstruction is described. Iterative image reconstruction refers to the methods for the image reconstruction. Multimodality refers to the ability of using different types of input parameters for an image. For example, in examples not part of the claimed invention, CT and MRI data may be used to reconstruct a MRI image; reflection data may be used to reconstruct a speed of sound image; or a first bandwidth range of data may be used to reconstruct an image covering a larger or different bandwidth range of data. The resulting output that may be of different modality or characteristics is referred to as "synthetic" output, which may be raw data or images. Synthetic information is information of a type that emulates the real information of that type but is either made from a different type of information or is of the same type but with different aspects (e.g., frequencies).

The ability to reconstruct images from suboptimal inputs as provided by certain embodiments of the described systems and techniques for "efficient" image reconstruction is highly advantageous. For example, certain embodiment of the described systems and techniques can allow for data received from cheaper, older, or more general machines to be used to reconstruct useful and detailed images. In some cases, mobile scanners can be used, and the more complex reconstruction can be carried out on separate systems such as described herein. The mobile scanners may be reflection ultrasound scanners and/or transmission ultrasound scanners with transmission frequencies above a certain threshold (e.g., starting between 800 kHz and 1.5 MHz). Transmission ultrasound scanners with larger frequency ranges (e.g., starting at lower frequencies such as 300 kHz) generally produce clearer and higher quality images; however, such transmission ultrasound scanners tend to require unconventional, finer or larger hardware to collect the transmission data, which often results in a more expensive machine. The described systems and techniques enable scanners of lower bandwidth to produce adequate images (which may be clearer or appear higher quality than what is usually able to be reconstructed from the data), which can further support availability of ultrasound images in a wide variety of applications and environments.

Indeed, in some cases, the described methods can be used to produce very rough scans, from relatively limited amount of data/projections, in a short period of time. This is especially useful as a real-time display while scanning (e.g., "scout scan") in order to aid in patient positioning. The scan can be cancelled in short order if the patient positioning is inadequate, and this could be determined quickly - far before the image would have been fully acquired and/or processed through more conventional means.

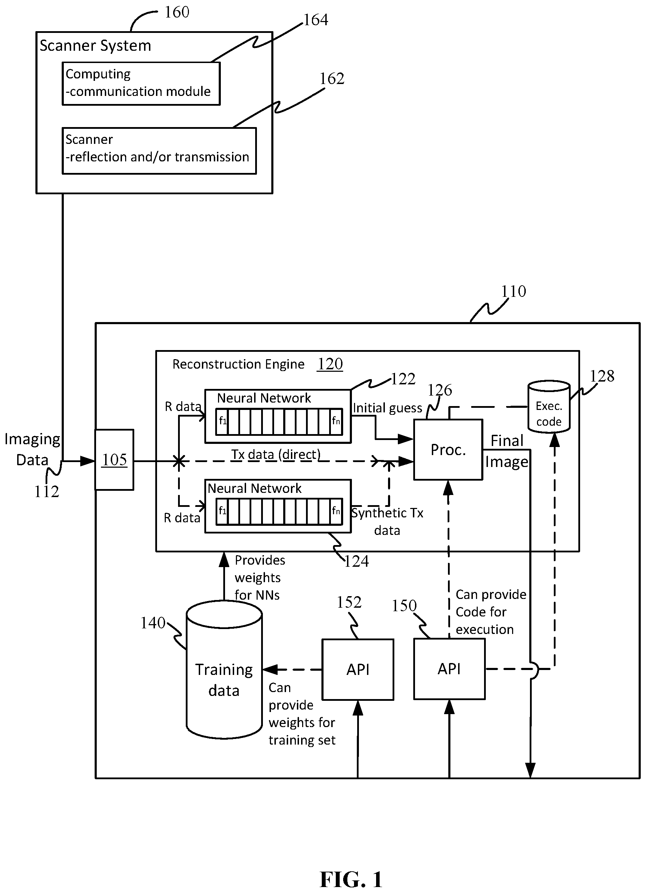

Figure 1 illustrates an operating environment for machine learning based image reconstruction. Referring to Figure 1, the operating environment can include an image reconstruction system **110.** The system **110** can include a communication interface **105** that can receive imaging data from a scanner system **160** and a reconstruction engine **120.** The system **110** can further include a storage device **140** containing training data for a neural network of reconstruction engine **120** (e.g., neural networks **122, 124),** and one or more APIs **150, 152.**

The system **110** can be remote, cloud-based, or locally operated. Similarly, the components of the system can exist in the same space or some or all can be hosted in different locations (e.g., distributed). In some cases, inputted data can be streamed in real-time from a scanner system **160.** In some cases, input data can be batch received or obtained post scan, for example, directly from a scanner system **160** or when the scanner system **160** transmits the input data to an intermediary system, which provides or makes available the data to the system **110** after completion of a scan (or parts of the scan). Interface **105** may be any suitable communications interface that can communicate wired or wirelessly with scanner system **160** directly or indirectly.

For ultrasound implementations, the system **110** can take in, depending on implementation, ultrasound data as input. Ultrasound data can include transmission data providing speed-of-sound and attenuation information and reflection data providing reflection information. Raw data or images may be received. The system **110** can, in other non-claimed implementations, receive as input various other image datatypes, such as CT or MRI. The reconstruction engine **120** can be the part of the system that transforms the input (e.g., any of the image data described above) into the output of a reconstructed image. It should be understood that the system **110** is capable of using one imaging modality datatype (e.g., MRI) and output another synthetic imaging modality datatype (e.g., ultrasound) with the use of paired data, as is explained further below.

One method of machine learning based image reconstruction uses neural networks. Accordingly, the reconstruction engine **120** can include at least one neural network (such as one, both, or more than the two neural networks **122, 124** illustrated in Figure 1), a processor **126,** and a computer storage system **128** storing executable code, for example a reconstruction algorithm, that can be executed by processor **126.**

The one or more neural networks **122, 124** can be Generative Adversarial Networks (GANs). Accordingly, at least one neural network includes a GAN-based algorithm that receives at least a component of the imaging data from the communication interface **105.**

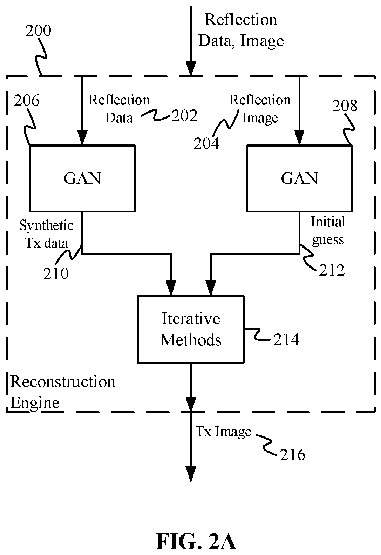

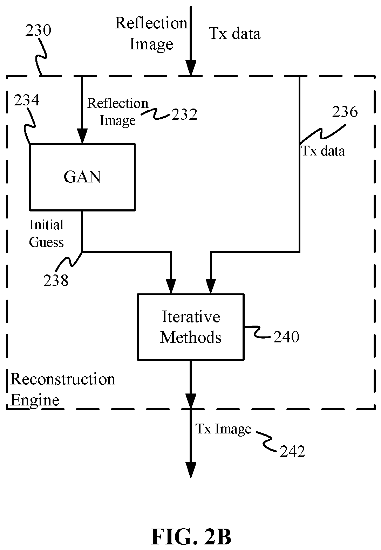

The inputs to the reconstruction engine **120** as a whole can be directly input to the neural networks (e.g., neural networks **122, 124).** In some cases, the inputs to the neural networks **122, 124** undergo data normalization and/or conversion from 3D volumes into 2D slices. Example implementations of reconstruction engines can be seen in Figures 2A and 2B.

The neural networks **122, 124** can utilize feature sets that serve to assist in transforming one or more images and/or datasets into another image and/or dataset. Features such as interfaces where the tissue/matter changes within a reflection image - for instance, skin and connective tissue elements such as Cooper's ligaments - have a large impact on defining boundaries within the generated speed-of-sound images (see also U.S. Application No. 15/836,576, entitled "Color Coding An Image for Identifying Anatomy Using Quantitative Transmission Ultrasound Tomography", for identification of features. Particularly when a cyclical neural network, such as a CycleGAN is used, features such as interfaces between skin and water, dense and fatty tissue, cysts and solids, and physical tissue deformations in the presence of medical tape can be useful in generating reflection images (see also U.S. Application No. 15/836,576, entitled "Color Coding An Image for Identifying Anatomy Using Quantitative Transmission Ultrasound Tomography", for identification of features).

Processor **126** can receive the output of the neural network(s) **122, 124** (and additionally, in some cases, possibly a direct input to the system **110,** which may or may not have been used by reconstruction engine **120).** Via execution of executable code, the processor **126** can perform image reconstruction, for example, via iterative methods for image reconstruction.

In the present invention, the at least one neural network includes two neural networks (e.g., neural networks **122, 124)** and the imaging data received via interface **105** is reflection data and a reflection image. The described neural networks can be used to estimate a transmission image from a reflection image, which serves a similar purpose to low frequency transmission data in providing a starting point for image reconstruction to be tuned by higher frequencies.

One neural network **124** receives the reflection data and generates a set of synthetic transmission data from the reflection data. The other neural network **122** receives the reflection image and generates an intermediate synthetic transmission image from the reflection image. The instructions for performing image reconstruction, when executed by the processor **126,** then direct the processor **126** to at least: perform iterative methods on the set of synthetic transmission data and the intermediate synthetic transmission image until a desired accuracy is reached, and upon reaching the desired accuracy, output a final synthetic transmission image.

A similar method can also be used if both reflection and transmission data are available to produce a superior image by using the latest image produced in each iteration to perform a check on the corresponding other image. For example, the imaging data can be high frequency transmission data and a reflection image. The at least one neural network (e.g., neural network **122)** receives the reflection image and generates an intermediate synthetic transmission image from the reflection image. The instructions for performing image reconstruction, when executed by the processor **126,** then direct the processor **126** to at least: perform iterative methods on the high frequency transmission data and the intermediate synthetic transmission image until a desired accuracy is reached, and upon reaching the desired accuracy, output a final synthetic transmission image.

As can be seen from the examples illustrated above, the inputs of the processor **126** can be an initial guess of the image that is provided from the output of one of the neural network(s) **122, 124,** transmission data, and/or reflection data. The output of the processor **126** can be the final image.

Example methods for image reconstruction can incorporate inverse scattering techniques, such as but not limited to the techniques described in "3-D Nonlinear Acoustic Inverse Scattering: Algorithm and Quantitative Results" (Wiskin et al., IEEE Transactions on Ultrasonics, Ferroelectrics, and Frequency Control, Vol. 64, No. 8, August 2017).

In some cases, image generation/reconstruction can use the neural network(s) to selectively remove and/or replace relatively higher noise (or low signal to noise ratio) image data at certain frequencies and/or viewing angles.

In some cases, the neural network(s) can be tuned to selectively improve and/or enhance the image quality of a certain tissue type or a region of interest. For example, use of a neural-network can selectively enhance the visibility of region with microcalcifications. In some of these cases, image quality in the rest of the breast tissue may become worse. In these cases, the enhanced visibility in the region with the microcalcifications may be merged with the original image (minus the corresponding region with the microcalcifications in the original image) to create an enhanced image. However, in some cases, the image quality in the rest of the breast tissue does not become worse and therefore does not need to be merged with the original image.

In some cases, system **110** can include an application programming interface (API) **150** enabling third parties to provide reconstruction algorithms (e.g., in the form of executable code) used by the reconstruction engine (e.g., processor **126).** This API **150** can be considered an image reconstruction selector API. In some cases, the image reconstruction selector API can be used to receive an image reconstruction algorithm for execution by the processor. In some cases, the image reconstruction selector API can be used to select an existing available image reconstruction algorithm for execution by the processor. In some cases, the image reconstruction selector API supports extensibility of the reconstruction engine for use in image reconstruction of the data/images generated by the neural networks by other parties.

The storage device **140** can store training data used to generate weights for the neural network(s) **122, 124** of the reconstruction engine **120.** The training data can be the sources of the weighting for the features (e.g., interfaces such as skin and water, dense and fatty tissue, cysts and solids, and physical tissue deformations in the presence of medical tape, and/or skin and connective tissue elements such as Cooper's ligaments) used by the neural networks in the system.

A variety of imaging modalities may be used as input data to teach the GAN-based algorithm to generate speed or reflection images. Such modalities include, but are not limited to, Transmission Ultrasound, Reflection Ultrasound, CT, MRI, PET, X-ray, SPECT, X-ray Mammography or any combination of multi-modalities. The system can create final images of these datatypes as the outputs. It may be desirable for such input data (i.e., training sets) to be obtained from scanning systems capable of corelating two or more imaging modalities (e.g., provide "paired data"). The correlation may be in terms of spatial and/or temporal dimensions between the provided paired data.

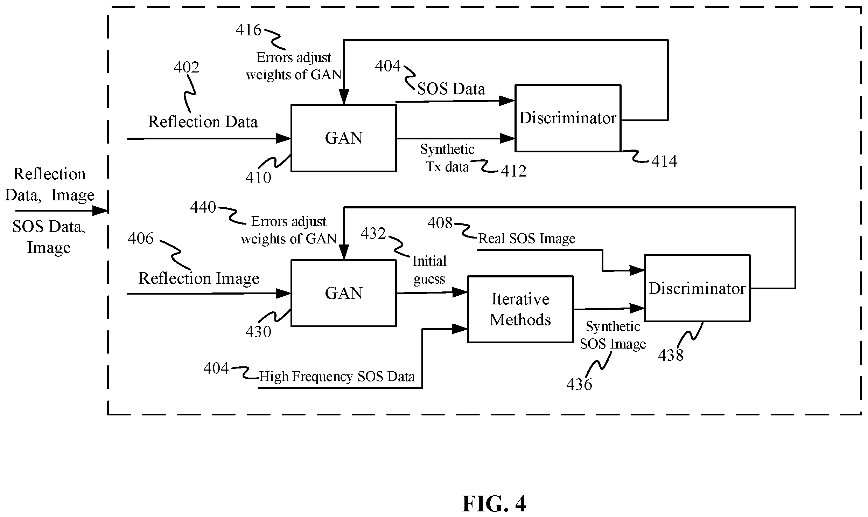

The training data itself can either be sourced externally or produced internally. In some cases, the output of the reconstruction engine **120** may be fed back to the system for self-learning. A possible methodology for obtaining training data from paired data is described in Figure 4. In some cases, system **110** includes an API **152** for submitting images for use to train the neural networks **122, 124** of the reconstruction engine **120** or possibly for direct implementation of weights for the neural networks **122, 124.** This API **152** can be considered a training data API. The training data API receives training data or weights for use in training the neural network.

In addition to APIs **150, 152,** other APIs may be available. An API is an interface implemented by a program code component or hardware component (hereinafter "API-implementing component") that allows a different program code component or hardware component (hereinafter "API-calling component") to access and use one or more functions, methods, procedures, data structures, classes, and/or other services provided by the API-implementing component. An API can define one or more parameters that are passed between the API-calling component and the API-implementing component.

The API is generally a set of programming instructions and standards for enabling two or more applications to communicate with each other and is commonly implemented over the Internet as a set of Hypertext Transfer Protocol (HTTP) request messages and a specified format or structure for response messages according to a REST (Representational state transfer) or SOAP (Simple Object Access Protocol) architecture.

The data used in the system **110** can be collected via a device such as scanner system **160,** which can also be considered an optional part of the system. The scanner system **160** can be a machine that includes a scanner **162** that takes ultrasound, MRI, or CT readings. If the scanner system **160** is an ultrasound scanner, reflection data and, optionally, transmission data can be collected. For transmission ultrasound scanner implementations, the scanner system **160** can take readings in at least the 800 kHz to 1.5 MHz range. The scanner system **160** can include a communication module **164** for communicating with system **110** (or an intermediate system) to provide image data to the system **110.** The scanner system **160** can also optionally include a storage system for storage of scanned data if the data is not streamed live.

Figures 2A and 2B illustrate example reconstruction engine configurations. Figure 2A illustrates an implementation according to the claimed invention using only reflection data, and Figure 2B illustrates a non-claimed implementation using reflection data along with transmission data. In both examples, the reconstruction engine can perform a method including receiving imaging data; generating, using at least one GAN-based neural network, intermediate synthetic transmission data from at least a component of the imaging data; performing iterative methods on at least the intermediate synthetic transmission data until a desired accuracy is reached; and upon reaching the desired accuracy, outputting final synthetic transmission data.

Figure 2A illustrates a reconstruction engine **200** for an input set of imaging data that includes only reflection data (which can be in the form of both raw reflection data and a reflection image) that generates intermediate synthetic transmission data of both a set of synthetic transmission data and an initial guess for a transmission image and outputs a synthetic transmission image as the final synthetic transmission data.

A reconstruction engine **200** that includes only reflection data as input and outputs a synthetic transmission image is useful because a reflection data input set represents something of a barebones setup - a system that produces only reflection data can be cheaper, as an entire sensor array is excluded. Alternatively, a reconstruction engine **200** that includes only reflection data as input and outputs a synthetic transmission image could be used for diagnostic purposes in the 10 mm region (e.g., for imaging areas where the angle for that type of imaging modality is difficult to support transmission through the tissue), as transmission data is difficult to collect at this scale. For example, it can be difficult to capture information in the interface from a patient's chest cavity to the patient's breast.

The reflection image **204** can be created through traditional means (prior to receipt at the reconstruction engine **200** or by a component of the reconstruction engine that is not shown in the figure), and the transmission data that one otherwise might not be able to obtain could be constructed through this process. A neural network **206** can receive the reflection data **202** and output a set of synthetic transmission data **210** for the same scanned object, while another neural network **208** can receive the reflection image **204** as input and can output an initial guess for the transmission image **212.** The processor (e.g., processor **126** of Figure 1) can receive the initial guess for the transmission image **212** and the set of synthetic transmission data **210,** execute the code (e.g., in the form of a reconstruction algorithm) that performs the iterative methods **214,** and produce a final transmission image **216.**

Accordingly, a reconstruction method can include generating, using a first GAN-based neural network, an intermediate set of synthetic transmission data from the reflection data; generating, using a second GAN-based neural network, an intermediate synthetic transmission image (the initial guess) from the reflection image; and performing iterative methods on the intermediate set of the synthetic transmission data and the intermediate synthetic transmission image until a desired accuracy is reached in order to output a final synthetic transmission image.

Figure 2B illustrates a reconstruction engine **230** for an input set of imaging data that includes transmission data in addition to the reflection data (which can be in the form of either raw reflection data that is then used to generate a reflection image through traditional means or is received in the form of the reflection image) and that generates intermediate synthetic transmission data of an initial guess for a transmission image and outputs a transmission image as the final synthetic transmission data. The transmission data can possibly be limited to only relatively high frequencies, such as starting at 800 kHz to 1.5 MHz. This can be useful because low frequency data requires finer and often more expensive or bulkier equipment. The methodology as a whole has advantages over existing implementations, even when the low frequency transmission data is available. Since the reflection image can be used to create an initial estimate for the transmission image fairly quickly (e.g., 10 seconds, 30 seconds, 1 minute, 5 minutes; for example, an estimate may be possible within 1-2 minutes for a typical breast size), the amount of time the system takes to reach a final estimate is cut down. To implement this methodology, one neural network **234** can receive the reflection image **232** as input and output an initial guess for the transmission image **238.** This image can be used, along with the received transmission data **236,** as an input for the processor (e.g., processor **126** of Figure 1) that executes the code that performs the iterative methods **240.** The output can be the final transmission image **242.**

Accordingly, a reconstruction method can include generating, using one GAN-based neural network, an intermediate synthetic transmission image (the initial guess) from the reflection image; and performing iterative methods on the intermediate synthetic transmission image and received transmission data until a desired accuracy is reached in order to output a final synthetic transmission image.

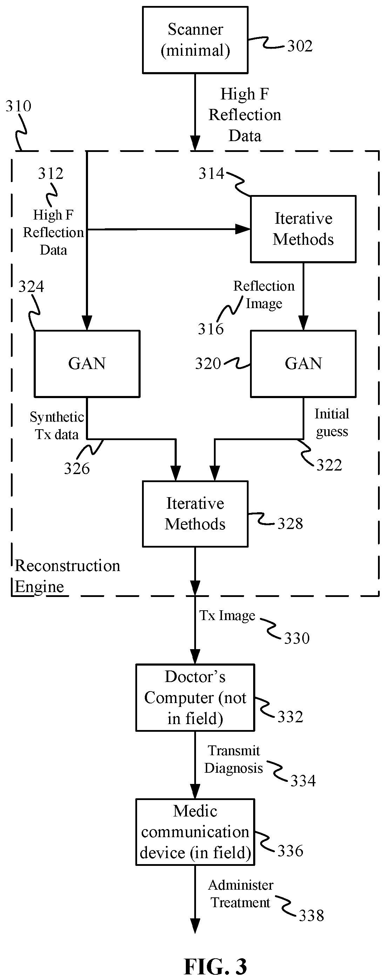

Figure 3 illustrates an example scenario using machine learning based image reconstruction. The environment described is a combat scenario, where medics can quickly and accurately provide treatment to soldiers using ultrasound analysis with minimal equipment. A soldier might be injured in the field, prompting a medic to use a simple ultrasound device **302.** This device may only gather reflection data **312,** as transmission data requires an extra receiver, and may only acquire high frequency data, as low frequency data requires a more complicated and often less portable device. This data can be collected and sent to the cloud (or to a server implementing the described recognition engine located nearby or remotely). A reconstruction engine **310,** such as described with respect to **120** of Figure 1, **200** of Figure 2A, or **230** of Figure 2B, can use the high-frequency reflection data **312** received directly from the ultrasound device **302** or indirectly (e.g., via a computing system that can communicate over a network) as an input to perform iterative methods **314** to generate an intermediate reflection image **316.** Then, a neural network **320** generates an initial guess for the transmission image **322** using the intermediate reflection image **316.** A separate neural network **324** can receive the high-frequency reflection data **312** as an input to produce a set of synthetic transmission data **326.** The synthetic transmission data **326** and the initial guess for the transmission image **322** are taken as inputs to an iterative method **328,** which produces the finalized transmission image **330.** An army doctor, not in the field, can receive these images on the doctor's computer **332.** The doctor can make a recommendation (e.g., diagnosis) for treatment based on the images and forward **334** the recommendation to the medic (e.g., via a communication device **336),** who can carry out **(338)** the instructions, possibly without ever leaving the field. Communication device **336** can be a phone, computer, or other device that can receive audio or visual input.

Figure 4 illustrates an example process for training a feature set of a neural network using paired data. The process can be implemented within an image recognition system such as image recognition system **110** described with respect to Figure 1. In some cases, one or more discriminators can be included for use in training the one or more neural networks **222, 224.** Turning to the example in Figure 4, for a certain dataset, there can exist both reflection and transmission data and images for a 3D volume position corresponding to a certain scanned object. The set of transmission and reflection data at each individual position in the 3D image space can be considered a set of paired data, as each (spatial and/or temporal) point in the reflection data can correspond to a single point in the transmission data. For transforming reflection data into synthetic transmission data, a neural network **410** can take in reflection data **402** as an input. The output synthetic transmission data **412** can be compared against the input transmission data **404** using a discriminator **414** that outputs a set of errors **416** that can be used to adjust the weights of the neural network. This process can occur iteratively, until a neural network that adequately constructs a set of synthetic data that is sufficiently close (e.g., within a range of error that comes to a resolution/resolves, etc.) to the corresponding paired data. The neural network **410** can also be trained across multiple datasets (e.g., using paired data from different imaging modalities) to find a set of weights that is most useful across all possible sets.

For training a neural network to transform a reflection image into a transmission image, a similar process can be employed. The neural network **430** to be trained can take the reflection image **406** as an input and output an initial guess **432** of a transmission image. A set of iterative methods **434** can receive the transmission data **404** (possibly including only the high frequency data) and the initial guess **432** of the transmission image to output a synthetic transmission image **436.** A discriminator **438** can compare the synthetic transmission image to the real transmission image **408** and produce a set of errors **440** that can be used to adjust the weights of the neural network. This process can occur iteratively, until a neural network that adequately constructs a synthetic transmission image that is sufficiently close to the corresponding paired image. The neural network **430** can also be trained across multiple datasets to find a set of weights that is most useful across all possible sets.

Another method of training can involve a GAN-based algorithm called CycleGAN. CycleGAN can be used to generate speed images from reflection images and vice-versa. Based on paired transmission and reflection images, CycleGAN can learn how to convert a speed image into a reflection image, and a reflection image into a speed image, at the same time. The algorithm can employ a concept where a discriminator function is used to minimize the error in the conversion from speed, to reflection, and back to speed again. The source of this error can be determined by the discriminator and the weights and features for the CycleGAN can be modified to reduce the error in future iterations. Further, at the same time, an attempt can be made to minimize the error in the conversion from reflection, to speed, and back to reflection again.

The training of the neural network can occur in two dimensions or three dimensions. Training in three dimensions can allow for the neural network to better differentiate between tissues as well as learn about tissue connectivity. For instance, adjacent voxels with similar values can be searched in three dimensions to more easily distinguish between tissues. This approach can improve differentiation between anatomy, for example, between ligaments and other elements that may have similar values, but not similar shapes.

Using paired training leaves open the possibility for a very strong and flexible system. If two systems have data that can be paired and are related at all, it is possible to train the neural network transform one into another. If the paired data exists at runtime - in other words, if the system is presented with paired data such as ultrasound and MRI - the two can be used together to refine the model more quickly and accurately than if the two were to be run by neural networks without paired data, as you create two models for testing in a single cycle.

The use of paired data can also be useful when using exclusively ultrasound data. If both reflection and transmission data is present, the system can compare the results of each iteration in the iterative methods to the results of the other iteration. The comparison can lead to improving the speed of the formation of the final image by comparing the two and using the commonalities as a stronger basis for the initial image used for the next iteration. Accordingly, a method for training a neural network can include receiving training imaging data, the imaging data comprising paired transmission data and reflection data; generating, using a GAN-based neural network, synthetic transmission data from the reflection data; comparing transmission data that is paired with the reflection data with the synthetic transmission data to determine a set of errors; and adjusting weights of the GAN-based neural network using the set of errors.

In some cases, in addition to or as an alternative, the method can include receiving paired transmission images and reflection images, generating using a GAN-based neural network, an initial guess of a synthetic transmission image from a reflection image; performing iterative methods using speed of sound data corresponding to a transmission image that is paired with the reflection image and the initial guess of the synthetic transmission image until a desired accuracy is reached; upon reaching the desired accuracy, outputting a final synthetic transmission image; comparing the transmission image that is paired with the reflection image with the final synthetic transmission image to determine a set of errors; and adjusting the weights of the GAN-based neural network using the set of errors. Initial weights for the neural networks can be received via a training data application programming interface such as described with respect to API **152** of Figure 1.

Although the subject matter has been described in language specific to structural features and/or acts, it is to be understood that the subject matter defined in the appended claims is not necessarily limited to the specific features or acts described above. Rather, the specific features and acts described above are disclosed as examples of implementing the claims and other equivalent features and acts are intended to be within the scope of the claims, provided they are part of the scope of the claims.

## Claims

1. A system (110) comprising:
a communication interface (105) configured to receive ultrasound imaging data from a scanner system (160,302);
a reconstruction engine (120,200,310) comprising:
two GAN-based neural networks (122,124,206,208,320,324,410,430) configured to receive, respectively, at least a component of the ultrasound imaging data from the communication interface (105);
a processor (126); and
storage media (128) storing instructions for performing image reconstruction using outputs of the two neural network (122,124,206,208,320,324,410,430) when executed by the processor (126),
wherein:
the ultrasound imaging data is reflection data (202,312,402) and an ultrasound reflection image (204,316,406);
one of the two neural networks (124,206,324,410) is configured to receive the reflection data (202,312,402) and to generate a set of synthetic transmission data (210,326,412) from the reflection data (202,312,402); and
the other one of the two neural networks (122,208,320,430) is configured to receive the ultrasound reflection image (204,316,406) and to generate an intermediate synthetic ultrasound transmission image (212,322,432) from the ultrasound reflection image (204,316,406),
wherein the instructions for performing image reconstruction, when executed by the processor (126), direct the processor (126) to at least:
perform iterative methods (214,328) on the set of synthetic transmission data (210,326,412) and the intermediate synthetic ultrasound transmission image (212,322,432) until a desired accuracy is reached; and
upon reaching the desired accuracy, output a final synthetic ultrasound transmission image (216,330).

2. The system of claim 1, further comprising:
an image reconstruction selector application programming interface (150), wherein the image reconstruction selector application programming interface (150) receives an image reconstruction algorithm for execution by the processor (126); or
a training data application programming interface (152), wherein the training data application programming interface (152) receives training data or weights for use in training the neural networks (122,124,206,208,320,324,410,430).

3. The system of claim 1, wherein the reconstruction engine (120) further comprises a discriminator (414) and a repository of corresponding reflection and transmission data;
wherein the discriminator (414) compares transmission data (404) corresponding to the reflection data (402) to the synthetic transmission data (412) and outputs a set of errors (416) that are used to adjust weights in the GAN-based neural network (410) generating the set of synthetic transmission data.

4. The system of claim 1, further comprising a discriminator (438) and a repository of corresponding ultrasound reflection and transmission images;
wherein the discriminator (438) compares an ultrasound transmission image (408), corresponding to the ultrasound reflection image (406), to a synthetic ultrasound transmission image determined based on the intermediate synthetic ultrasound transmission image, and outputs a set of errors that are used to adjust weights in the GAN-based neural network (430) generating the intermediate synthetic ultrasound transmission image.

5. The system of claim 1, wherein the the at least two GAN-based neural networks (122,124,206,208,320,324,410,430) are configured to identify features within the ultrasound imaging data.

6. A computer-implemented method comprising:
receiving ultrasound imaging data;
generating, using two GAN-based neural networks (122,124,206,208,320,324,410,430), intermediate synthetic transmission data (210,326,412,212,322,432) from at least a component of the ultrasound imaging data;
performing iterative methods (214,328) on at least the intermediate synthetic transmission data (210,326,412,212,322,432) until a desired accuracy is reached; and
upon reaching the desired accuracy, outputting final synthetic transmission data,
wherein components of the ultrasound imaging data are reflection data (202,312,402) and an ultrasound reflection image (204,316,406); and
wherein generating, using the two GAN-based neural networks (122,124,206,208,320,324,410,430), the intermediate synthetic transmission data (210,326,412, 212,322,432) from the components of the ultrasound imaging data comprises:
generating, using a first GAN-based neural network (124,206,324,410) of the two GAN-based neural networks (122,124,206,208,320,324,410,430), an intermediate set of synthetic transmission data (210,326,412) from the reflection data (202,312,402); and
generating, using a second GAN-based neural network (122, 208, 320, 430) of the two GAN-based neural networks (122,124,206,208,320,324,410,430), an intermediate synthetic ultrasound transmission image (212,322,432) from the ultrasound reflection image (204,316,406),
wherein performing iterative methods on at least the intermediate synthetic transmission data until a desired accuracy is reached comprises performing iterative methods (214,328) on the intermediate set of the synthetic transmission data (210,326,412) and the intermediate synthetic ultrasound transmission image (212,322,432) until a desired accuracy is reached; and the final synthetic transmission data is a final synthetic ultrasound transmission image (216,330).

7. The method of claim 6, further comprising receiving, via an image reconstruction selector application programming interface (150), an image reconstruction algorithm for performing the iterative methods (214,328) on at least the intermediate synthetic transmission data (210,326,412, 212,322,432) until the desired accuracy is reached.

## Patentansprüche

1. System (110), umfassend:
eine Kommunikationsschnittstelle (105), die dazu konfiguriert ist, Ultraschall-Bildgebungsdaten von einem Scanner System (160, 302) zu empfangen;
eine Rekonstruktionsengine (120, 200, 310), umfassend:
eine Rekonstruktionseinheit (120, 200, 310), umfassend:
Zwei auf GANs basierende neuronale Netze (122, 124, 206, 208, 320, 324, 410, 430), die dazu konfiguriert sind, jeweils zumindest eine Komponente der Ultraschall-Bildgebungsdaten von der Kommunikationsschnittstelle (105) zu empfangen;
einen Prozessor (126); und
Speichermedium (128), das Anweisungen zur Bildrekonstruktion, unter Verwendung der Ausgaben der beiden neuronalen Netze (122, 124, 206, 208, 320, 324, 410, 430), speichert, wenn diese vom Prozessor (126) ausgeführt werden.
wobei es sich bei den Ultraschall-Bildgebungsdaten um Reflexionsdaten (202, 312, 402) und ein Ultraschall-Reflexionsbild (204, 316, 406) handelt;
Eines der beiden neuronalen Netze (124, 206, 324, 410) ist dazu konfiguriert, die Reflexionsdaten (202, 312, 402) zu empfangen und aus den Reflexionsdaten (202, 312, 402) einen Satz synthetischer Transmissionsdaten (210, 326, 412) zu erzeugen; und
das andere der beiden neuronalen Netze (122, 208, 320, 430) ist dazu konfiguriert, das Ultraschall-Reflexionsbild (204, 316, 406) zu empfangen und aus dem Ultraschall-Reflexionsbild (204, 316, 406) ein synthetisches Ultraschalltransmission-Zwischenbilds (212, 322, 432) zu erzeugen,
wobei die Anweisungen zur Durchführung der Bildrekonstruktion, wenn sie vom Prozessor (126) ausgeführt werden, den Prozessor (126) dazu anweisen, zumindest:
iterative Verfahren (214, 328) auf den Satz synthetischer Transmissionsdaten (210, 326, 412) und das synthetische Ultraschalltransmission-Zwischenbild (212, 322, 432) anwenden, bis eine gewünschte Genauigkeit erreicht ist; und
bei Erreichen der gewünschten Genauigkeit, wird ein abschließendes synthetisches Ultraschaltransmissionsbild (216, 330) ausgegeben.

2. System nach Anspruch 1, ferner umfassend:
Eine Anwendungsprogrammierschnittstelle (150) für die Auswahl der Bildrekonstruktion, wobei die Anwendungsprogrammierschnittstelle (150) für die Auswahl der Bildrekonstruktion einen Bildrekonstruktionsalgorithmus zur Ausführung durch den Prozessor (126) empfängt; oder
eine Anwendungsprogrammierschnittstelle (152) für Trainingsdaten, wobei die wobei die Anwendungsprogrammierschnittstelle (152) für Trainingsdaten die Trainingsdaten oder Gewichtungen zur Verwendung beim Training der neuronalen Netze (122, 124, 206, 208, 320, 324, 410, 430) empfängt.

3. System nach Anspruch 1, wobei die Rekonstruktionsengine (120) ferner einen Diskriminator (414) sowie ein Repository mit entsprechenden Reflexions- und Transmissionsdaten umfasst;
wobei der Diskriminator (414) Transmissionsdaten (404), die den Reflexionsdaten (402) entsprechen, mit den synthetischen Transmissionsdaten (412) vergleicht und einen Satz von Fehlern (416) ausgibt, die dazu verwendet werden, Gewichtungen in dem GAN-basierten neuronalen Netzwerk (410) anzupassen, welches den Satz synthetischer Transmissionsdaten erzeugt.

4. System nach Anspruch 1, ferner einen Diskriminator (438) und ein Repository von entsprechender Ultraschall-Reflexions- und -Transmissionsbildern umfassend;
wobei der Diskriminator (438) ein Ultraschall-Transmissionsbild (408), das dem Ultraschall-Reflexionsbild (406) entspricht, mit einem synthetischen Ultraschall-Transmissionsbild vergleicht, das auf der Grundlage des synthetischen Ultraschalltransmission-Zwischensbilds bestimmt wurde, und einen Satz von Fehlern ausgibt, die dazu verwendet werden, Gewichtungen in dem GAN-basierten neuronalen Netzwerk (430) anzupassen, welches das synthetische Ultraschalltransmission-Zwischensbild erzeugt.

5. System nach Anspruch 1, wobei die mindestens zwei GAN-basierten neuronalen Netze (122, 124, 206, 208, 320, 324, 410, 430) dazu konfiguriert sind, Merkmale innerhalb der Ultraschallbilddaten zu identifizieren.

6. Computerimplementiertes Verfahren, umfassend:
Empfangen von Ultraschallbilddaten;
Erzeugen synthetischer Transmission-Zwischendaten (210, 326, 412, 212, 322, 432) - unter Verwendung von GAN-basierten neuronalen Netzen (122, 124, 206, 208, 320, 324, 410, 430) - aus zumindest einem Teil der Ultraschall-Bildgebungsdaten;
Durchführen iterativer Verfahren (214, 328) an zumindest den synthetischen Transmission-Zwischendaten (210, 326, 412, 212, 322, 432), bis eine gewünschte Genauigkeit erreicht ist; und
nach Erreichen der gewünschten Genauigkeit ausgeben der endgültigen synthetischen Transmissionsdaten,
wobei es sich bei Komponenten der Ultraschall-Bildgebungsdaten um Reflexionsdaten (202, 312, 402) und ein Ultraschall-Reflexionsbild (204, 316, 406) handelt; und
wobei das Erzeugen der synthetischen Transmission-Zwischendaten (210, 326, 412, 212, 322, 432) aus den Komponenten der Ultraschall-Bildgebungsdaten unter Verwendung der beiden GAN-basierten neuronalen Netzwerke (122, 124, 206, 208, 320, 324, 410, 430) Folgendes umfasst:
Erzeugen, unter Verwendung eines ersten GAN-basierten neuronalen Netzes (124, 206, 324, 410) der zwei GAN-basierten neuronalen Netze (122, 124, 206, 208, 320, 324, 410, 430), (124, 206, 324, 410), eines Zwischensatzes synthetischer Transmissionsdaten (210, 326, 412) aus den Reflexionsdaten (202, 312, 402); und
Erzeugen, unter Verwendung eines zweiten GAN-basierten neuronalen Netzes (122, 208, 320, 430) der beiden GAN-basierten neuronalen Netze (122, 124, 206, 208, 320, 324, 410, 430), eines synthetischen Ultraschallzwischenbildes (212. 322, 432) aus dem Ultraschall-Reflexionsbild, (204, 316, 406),
wobei die Durchführung iterativer Verfahren - zumindest an den synthetischen Transmissionszwischendaten, bis eine gewünschte Genauigkeit erreicht ist - die Anwendung iterativer Verfahren (214, 328) auf den Zwischensatz der synthetischen Transmissionsdaten (210, 326, 412) sowie auf das synthetische Ultraschall-Transmissionszwischenbild (212, 322, 432) umfasst, bis eine gewünschte Genauigkeit erreicht ist; und wobei es sich bei den endgültigen synthetischen Transmissionsdaten um ein endgültiges synthetisches Ultraschall-Transmissionsbild (216, 330) handelt.

7. Verfahren nach Anspruch 6, ferner umfassend das Empfangen eines Bildrekonstruktionsalgorithmus über eine Anwendungsprogrammierschnittstelle (150) für die Auswahl von Bildrekonstruktionsverfahren (150), um die iterativen Verfahren (214, 328) auf zumindest die synthetischen Transmissionszwischendaten (210, 326, 412; 212, 322, 432) anzuwenden, bis die gewünschte Genauigkeit erreicht ist.

## Revendications

1. Un système (110) comprenant :
une interface de communication (105) configurée pour recevoir des données d'imagerie ultrasonore provenant d'un système de scanner (160,302) ;
un moteur de reconstruction (120,200,310) comprenant :
deux réseaux neuronaux GAN (122,124,206,208,320,324,410,430) configurés pour recevoir, respectivement, au moins une composante des données d'imagerie ultrasonore provenant de l'interface de communication (105) ;
un processeur (126) ; et
un support de stockage (128) stockant des instructions pour exécuter la reconstruction d'image à l'aide de sorties des deux réseaux neuronaux (122,124,206,208,320,324,410,430) quand elle est effectuée par le processeur (126),
dans lequel les données d'imagerie ultrasonore sont des données de réflexion (202,312,402) et une image de réflexion ultrasonore (204,316,406) ;
l'un des deux réseaux neuronaux (124,206,324,410) est configuré pour recevoir les données de réflexion (202,312,402) et pour générer un ensemble de données de transmission synthétiques (210,326,412) à partir des données de réflexion (202,312,402) ; et
l'autre des deux réseaux neuronaux (122,208,320,430) est configuré pour recevoir l'image de réflexion ultrasonore (204,316,406) et pour générer une image de transmission ultrasonore synthétique intermédiaire (212,322,432) à partir de l'image de réflexion ultrasonore (204,316,406),
dans lequel les instructions pour effectuer la reconstruction d'image, quand elles sont exécutées par le processeur (126), indiquent au processeur (126) d'exécuter au moins :
des méthodes itératives (214,328) sur l'ensemble de données de transmission synthétiques (210,326,412) et l'image de transmission ultrasonore synthétique intermédiaire (212,322,432) jusqu'à ce qu'une précision souhaitée soit atteinte ; et
une fois la précision souhaitée atteinte, sortir une image de transmission ultrasonore synthétique finale (216,330).

2. Le système de la revendication 1, comprenant en sus :
une interface de programmation d'application de sélecteur de reconstruction d'image (150), dans lequel l'interface de programmation d'application de sélecteur de reconstruction d'image (150) reçoit un algorithme de reconstruction d'image à exécuter par le processeur (126) ; ou
une interface de programmation d'application pour les données d'entraînement (152), dans lequel l'interface de programmation d'application pour les données d'entraînement (152) reçoit des données d'entraînement ou des poids à utiliser dans l'entraînement des réseaux neuronaux (122,124,206,208,320,324,410,430).

3. Le système de la revendication 1, dans lequel le moteur de reconstruction (120) comporte en sus un discriminateur (414) et un référentiel des données de transmission et de réflexion correspondantes ;
dans lequel le discriminateur (414) compare les données de transmission (404) correspondant aux données de réflexion (402) aux données de transmission synthétiques (412) et produit un ensemble d'erreurs (416) qui servent à ajuster les poids dans le réseau neuronal GAN (410) générant l'ensemble de données de transmission synthétiques (412).

4. Le système de la revendication 1, comprenant en sus un discriminateur (438) et un référentiel d'images de transmission et de réflexion ultrasonores correspondantes ;
dans lequel le discriminateur (438) compare une image de transmission ultrasonore (408), correspondant à l'image de réflexion ultrasonore (406), à une image de transmission ultrasonore synthétique déterminée sur la base de l'image de transmission ultrasonore synthétique intermédiaire, et produit un ensemble d'erreurs (416) qui servent à ajuster les poids dans le réseau neuronal GAN (430) générant l'image de transmission ultrasonore synthétique intermédiaire.

5. Le système de la revendication 1, dans lequel les au moins deux réseaux neuronaux GAN (122,124,206,208,320,324,410,430) sont configurés pour identifier des caractéristiques dans les données d'imagerie ultrasonore.

6. Un procédé mis en œuvre par ordinateur comprenant :
recevoir des données d'imagerie ultrasonore ;
générer, à l'aide de deux réseaux neuronaux GAN (122,124,206,208,320,324,410,430), des données de transmission synthétiques intermédiaires (210,326,412,212,322,432) à partir d'au moins une composante des données d'imagerie ultrasonore ;
exécuter des méthodes itératives (214,328) sur au moins les données de transmission synthétiques intermédiaires (210,326,412,212,322,432) jusqu'à ce qu'une précision souhaitée soit atteinte ; et une fois la précision souhaitée atteinte, sortir des données de transmission synthétiques finales,
dans lequel des composantes des données d'imagerie ultrasonore sont des données de réflexion (202,312,402) et une image de réflexion ultrasonore (204,316,406) ; et
dans lequel générer, à l'aide des deux réseaux neuronaux GAN (122,124,206,208,320,324,410,430), les données de transmission synthétiques intermédiaires (210,326,412,212,322,432) à partir des composantes des données d'imagerie ultrasonore comporte :
générer, à l'aide d'un premier réseau neuronal GAN (124,206,324,410) des deux réseaux neuronaux GAN (122,124,206,208,320,324,410,430), (124,206,324,410), un ensemble intermédiaire de données de transmission synthétiques (210,326,412) à partir des données de réflexion (202,312,402) ; et
générer, à l'aide d'un deuxième réseau neuronal GAN (122,208,320,430) des deux réseaux neuronaux GAN (122,124,206,208,320,324,410,430), une image de transmission ultrasonore synthétique intermédiaire (212,322,432) à partir de l'image de réflexion ultrasonore (204,316,406),
dans lequel l'exécution des méthodes itératives sur au moins les données de transmission synthétiques intermédiaires jusqu'à ce qu'une précision souhaitée soit atteinte comprend l'exécution des méthodes itératives (214,328) sur l'ensemble intermédiaire des données de transmission synthétiques (210,326,412) et l'image de transmission ultrasonore synthétique intermédiaire (212,322,432) jusqu'à ce qu'une précision souhaitée soit atteinte ; et les données de transmission synthétiques finales sont une image de transmission ultrasonore synthétique finale (216,330).

7. Le procédé de la revendication 6, comprenant en sus recevoir, via une interface de programmation d'application de sélecteur de reconstruction d'image (150), un algorithme de reconstruction d'image pour exécuter les méthodes itératives (214,328) sur au moins les données de transmission synthétiques intermédiaires (210,326,412,212,322,432) jusqu'à ce que la précision souhaitée soit atteinte.
